# EUROPÄISCHE PATENTSCHRIFT

(11) **EP 2 810 628 B1**
(45) Veröffentlichungstag und Bekanntmachung des Hinweises auf die Patenterteilung: **23.09.2015**
(21) Anmeldenummer: 14166544.8
(22) Anmeldetag: 30.04.2014
(51) Int. Cl.: A61F 11/00, A61B 17/00, A61B 17/16

(54) **Einweg-Knorpelschneider**
Disposable cartilage cutter
Dispositif de coupe de cartilage à usage unique

(30) Priorität: 06.06.2013 DE 202013102433 U
(43) Veröffentlichungstag der Anmeldung: 10.12.2014
(73) Patentinhaber: Heinz Kurz GmbH Medizintechnik, 72144 Dusslingen (DE)
(72) Erfinder: Steinhardt, Uwe, 72145 Hirrlingen (DE); Kurz, Heinz, 72144 Dusslingen (DE)
(74) Vertreter: Kohler Schmid Möbus Patentanwälte

(56) Entgegenhaltungen:
- EP-A1- 0 714 634
- EP-A1- 1 360 948
- US-A1- 2010 286 693
- US-A1- 2012 191 093

## Beschreibung

Die Erfindung betrifft eine medizinische Schneidevorrichtung zur Herstellung dünner Knorpelscheiben mit einem Vorrichtungskörper und einem Deckel, wobei eine erste Halteeinrichtung vorgesehen ist, die einen ersten Abschnitt mit einer auf der Oberseite des Vorrichtungskörpers angeordneten ersten Vertiefung aufweist, welche von einem ersten Begrenzungssteg ganz oder teilweise umrandet ist, und wobei die Schneidevorrichtung aus einem sterilisierbaren Material hergestellt ist.

Eine derartige Vorrichtung ist bekannt aus US 2010/0286693 A1, aus DE 20 2009 006 583 U1 beziehungsweise aus EP 2 249 139 A2.

Die Offenlegungsschrift US 2010/0286693 A1 offenbart den Gegenstand des Oberbegriffs von Anspruch 1.

### Hintergrund der Erfindung

Das Herausschneiden von dünnen körpereigenen Knorpelscheiben gleichmäßiger Dicken aus einem größeren, z.B. aus der Ohrmuschel, dem Tragus, dem knorpeligen Anteil der oberen Rippenknochen oder dem Nasenseptum entnommenen Knorpelstück ist für verschiedene medizinische Zwecke eine immer wieder erforderliche Tätigkeit. Beispielsweise kann es notwendig sein, spezielle Eigenschaften des Grundkörpers näher zu untersuchen, insbesondere unter einem Mikroskop. In der Hals-Nasen-Ohren-Heilkunde werden solche dünnen Knorpelscheiben aber auch bei vielen chirurgischen Eingriffen benötigt, etwa im Mittelohr-Bereich zum Abdecken einer Mittelohr-Prothese, zum Wiederaufbau der Gehörgangshinterwand oder zur plastischen Versorgung eines Trommelfell-Defekts. Auch bei vielen nasenchirurgischen Eingriffen werden derartige dünne Knorpelscheiben eingesetzt, um funktionelle oder ästhetische Korrekturen an der Nase durchzuführen.

In der EP 0 483 567 B1 ist eine Schneidevorrichtung beschrieben, mit welcher dünne Knorpelscheiben einer - in gewissen Grenzen - vorgebbaren Dicke aus deinem größeren Knorpelstück rasch, sicher und in gleichmäßiger Qualität herausgeschnitten werden können. Um unterschiedliche Dicken der erzeugten Knorpelscheiben erzielen zu können, müssen allerdings spezielle Distanzblättchen bekannter Dicke in die Schneidevorrichtung eingelegt werden. Diese Distanzblättchen müssen - ebenso wie die Schneidevorrichtung selbst - streng gereinigt und steril gehalten und vor jeder Operation eigens entsprechend behandelt werden, was einerseits zeitaufwändig und andererseits fehleranfällig ist. Bedenkt man, dass sich in einer durchschnittlichen HNO-Klinik drei bis vier Operationssäle befinden und zu Spitzenzeiten an einem Tag fünfzehn bis zwanzig Patienten operiert werden, so kann es sein, dass an einem Tag bis zu fünfzehn mal ein Knorpelschneider bereit gestellt werden muss. Dies ist für die Sterilgutversorgung eine große logistische Herausforderung.

Außerdem ist die Handhabung der Distanzblättchen nicht ganz einfach. So ist es zum Beispiel wegen ihrer geringen Größe nur bedingt möglich, sie ausreichend und gut erkennbar zu beschriften, was aber die Grundvoraussetzung dafür ist, dass während der Operation genau das Distanzblättchen mit der jeweils erforderlichen Größe bereit liegt. Auch das korrekte Einlegen und Fixieren der ziemlich kleinen Distanzblättchen in die Schneidevorrichtung erfordert einige Geschicklichkeit.

Um dünne Knorpelscheiben bestimmter unterschiedlicher Dicken in gleichmäßiger Qualität auch ohne die Verwendung der bekannten Distanzblättchen herstellen zu können, wird in der eingangs zitierten US 2010/0286693 A1 - zusätzlich zu den Merkmalen der eingangs beschriebenen gattungsgemäßen Schneidevorrichtung - vorgeschlagen, dass - wie auch bei der bekannten Schneidevorrichtung nach EP 0 483 567 B1 - die im ersten Arbeitsabschnitt auf der Oberseite des Vorrichtungskörpers angeordnete erste Vertiefung durch einen auf der Oberseite des Deckels angeordneten ersten Vorsprung verschließbar ist, wobei der erste seitliche Begrenzungssteg einen von einer Stirnseite des ersten Abschnitts her geführten, mit einem vorgegebenen ersten Abstand parallel zur Grundfläche der ersten Vertiefung verlaufenden ersten Führungsschlitz zum Einschieben einer Schneidklinge aufweist. Außerdem soll bei der Vorrichtung nach US 2010/0286693 A1 mindestens eine zweite Halteeinrichtung vorgesehen sein, die einen zweiten Abschnitt mit einer auf der Oberseite des Vorrichtungskörpers angeordneten zweiten Vertiefung aufweist, welche von einem zweiten Begrenzungssteg ganz oder teilweise umrandet und durch einen auf der Oberseite des Deckels angeordneten zweiten Vorsprung verschließbar ist, wobei der zweite seitliche Begrenzungssteg einen von einer Stirnseite des zweiten Abschnitts her geführten, mit einem vorgegebenen zweiten Abstand parallel zur Grundfläche der zweiten Vertiefung verlaufenden zweiten Führungsschlitz zum Einschieben einer Schneidklinge aufweist.

Da die Abstände zwischen dem jeweiligen Führungsschlitz und der entsprechenden Grundfläche der jeweiligen Vertiefung bei unterschiedlichen Halteeinrichtungen verschieden gewählt werden können, lassen sich mit dieser bekannten Schneidevorrichtung mit den unterschiedlichen Halteeinrichtungen Knorpelscheiben bestimmter unterschiedlicher Dicken herstellen, ohne dass dafür die beim früheren Stand der Technik unerlässlichen Distanzblättchen eingesetzt werden müssen. Weiter gibt eine Handhabung der neuen Schneidevorrichtung zwischen Daumen und Zeigfinger dem Operateur mehr Sicherheit beim eigentlichen Schneidevorgang, weil alle Teile aufgrund ihrer Geometrie und ihres Designs sicher und kontrolliert zueinander bewegt werden können.

Nachteilig bei dieser bekannten Schneidevorrichtung ist jedoch, dass Teile sich nur mit sehr großem Aufwand herstellen lassen. Gerade spritzgusstechnisch hergestellte Produkte haben die Eigenschaft, dass bei sehr dünnen Wandungen große Probleme entstehen können. Dies hat zur Folge, dass sich die Produkte aufgrund der finanziellen Aufwendungen in der Regel nicht wirtschaftlich realisieren lassen.

### Aufgabe der Erfindung

Der vorliegenden Erfindung liegt demgegenüber die Aufgabe zugrunde, eine gattungsgemäße medizinische Schneidevorrichtung der eingangs beschriebenen Art mit möglichst einfachen technischen Mitteln unaufwändig und kostengünstig dahin gehend zu verbessern, dass diese Nachteile vermieden werden, wobei aber die oben geschilderten Vorteile der bekannten Schneidevorrichtung gegenüber dem Stand der Technik beibehalten werden sollen. Insbesondere soll sichergestellt werden, dass der Schneidevorgang einfach, sicher und qualitativ hochwertig durchgeführt werden kann.

### Kurze Beschreibung der Erfindung

Diese Aufgabe wird durch den Gegenstand des unabhängigen Anspruchs gelöst. Weiterbildungen sind in den abhängigen Ansprüchen angegeben.

Diese komplexe Aufgabe wird auf ebenso überraschend einfache wie wirkungsvolle Weise dadurch gelöst, dass der Vorrichtungskörper einen Verbindungsabschnitt aufweist, an welchem der erste Arbeitsabschnitt unmittelbar starr anhängt, dass der Deckel ein Gegenstück zum Verbindungsabschnitt aufweist, an welchem ein erster Druckabschnitt unmittelbar starr anhängt, dass der erste Druckabschnitt eine federnd im ersten Druckabschnitt gehaltene zentrale erste Druckplatte aufweist, welche in einem Betriebszustand zur Herstellung dünner Knorpelscheiben der vom ersten Begrenzungssteg ganz oder teilweise umrandeten ersten Vertiefung gegenüberliegt, und dass die Abschnitte geometrisch derart gestaltet sind, dass im Betriebszustand das Gegenstück zum Verbindungsabschnitt arretierbar auf dem Verbindungsabschnitt aufliegt und der erste Druckabschnitt der ersten Vertiefung des ersten Arbeitsabschnitts mit einer durch die geometrische Formgebung des Verbindungsabschnitts sowie dessen Gegenstücks definierten, im Wesentlichen gleichmäßigen ersten Distanz dₐ gegenüberliegt, so dass ein zwischen dem ersten Arbeitsabschnitt und dem ersten Druckabschnitt verlaufender erster Führungsschlitz zum Einschieben einer Schneidklinge frei bleibt.

Über die federnde Aufhängung wird der Druck derart reguliert, dass in der Phase des Vorgangs eine Art Druckkompensation stattfindet, um einen gleichmäßigen Ablauf zu gewährleisten. Weiter hat der federngehaltene Druckabschnitt der Vorteil, dass der Anpressdruck sehr einfach aber genau dosiert werden kann. Über die Federung wird der direkte Druck kompensiert und für den Schneidevorgang vorteilhaft weitergegeben. Dadurch fällt es dem Anwender leicht, den Schneidevorgang sicher und qualitativ hochwertig durchzuführen.

Mit Hilfe der erfindungsgemäßen Schneidevorrichtung ist auch gewährleistet, dass eine Reinigung und erneute Sterilisation entfällt. Dies wird mehr und mehr wichtig, da die Verkeimung von chirurgischen Instrumenten ein immer größer werdendes Problem innerhalb des Klinikalltags darstellt.

### Bevorzugte Ausführungsformen der Erfindung

Ganz besonders bevorzugt sind Ausführungsformen der erfindungsgemäßen Schneidevorrichtung, bei denen eine Arretiervorrichtung vorgesehen ist, die den Vorrichtungskörper und den Deckel in einem zusammengeklappten Betriebszustand in einer fixen relativen Position zueinander hält und so eine sichere Bearbeitung der herzustellenden Knorpelscheibe sicherstellt.

Eine fertigungstechnisch sehr einfach herstellbare Weiterbildung dieser Ausführungsformen ist dadurch gekennzeichnet, dass die Arretiervorrichtung mindestens einen Zapfen und mindestens ein Nutenloch umfasst, in welchen der Zapfen arretierend eingesteckt werden kann.

Von großem praktischen Vorteil sind auch Ausführungsformen der Erfindung, bei welchen der Verbindungsabschnitt und sein Gegenstück geometrisch derart geformt sind, dass der Vorrichtungskörper und der Deckel nur in einer bestimmten vorgegebenen Richtung zusammensetzbar sind, so dass der Operateur nahezu "blind" arbeiten kann.

Diese Ausführungsformen lassen sich vorteilhaft noch dadurch weiterbilden, dass der Verbindungsabschnitt des Vorrichtungskörpers auf seiner die Vertiefungen tragenden Oberseite einen Aufnahmeraum aufweist, in welchen eine Erhöhung auf der die Druckplatten tragenden Oberseite des Deckels im Gegenstück passgenau eingeführt werden kann. Derartige Weiterbildungen der Erfindung sind auch relativ einfach herstellbar.

Besonders bevorzugt sind Varianten dieser Weiterbildungen, welche sich dadurch auszeichnen, dass die den Aufnahmeraum umschließende Wandung sowie die die Erhöhung umgebende Wandung jeweils ein Polygon gleicher Eckenzahl und Geometrie, vorzugsweise ein Dreieck oder ein Viereck, insbesondere ein Quadrat bilden, wodurch ein passgenaues, lediglich in einer definierten Relativposition mögliches Zusammenfügen von Vorrichtungskörper und Deckel für den zusammengeklappten Betriebszustand vereinfacht wird.

Weitere vorteilhafte Ausführungsformen der erfindungsgemäßen Schneidevorrichtung zeichnen sich dadurch aus, dass die Druckplatten federnd, insbesondere mittels vorzugsweise gekrümmten Stegelementen, in Ausnehmungen der Druckabschnitte eingehängt sind.

Um die zu verarbeitenden Knorpelstücke besser zu halten, können bei Ausführungsformen der Erfindung die Grundflächen der Vertiefungen und/oder die den Vertiefungen in einem zusammengeklappten Betriebszustand von Vorrichtungskörper und Deckel gegenüber liegende Flächen der Druckplatten aufgeraut, geriffelt oder genoppt sein.

Ganz besonders bevorzugt ist Klasse von Ausführungsformen der erfindungsgemäßen Schneidevorrichtung, bei denen die Schneidevorrichtung aus einem sterilisierbaren Kunststoff hergestellt ist. Damit lässt sich die Schneidevorrichtung ganz erheblich preisgünstiger herstellen als die üblichen Vorrichtungen aus Metall. Die Anlieferung zur Operation erfolgt dann in einer Sterilverpackung und die benutzte Schneidevorrichtung kann einfach entsorgt werden. Ein solches steril verpacktes Einmal-Produkt hat außerdem den Vorteil, dass nicht vor jeder Operation eine aufwändige Reinigung und Sterilisation der Schneidevorrichtung erfolgen muss und es minimiert sich auch das Risiko einer Infektion, dass bei einer Sterilgutversorgung nicht ausgeschlossen werden kann. Vorzugsweise wird bei Weiterbildungen dieser Klasse von Ausführungsformen die Schneidevorrichtung in einem Spritzgussverfahren hergestellt.

Eine weitere bevorzugte Klasse von Ausführungsformen der erfindungsgemäßen Schneidevorrichtung zeichnet sich dadurch aus, dass an den Arbeitsabschnitten des Vorrichtungskörpers und/oder an den entsprechenden Abschnitten des Deckels, welche die Vorsprünge tragen, Kennzeichnungen angebracht sind, die den durch die jeweilige Distanz dₐ, d_{b}, d_{c} vorgegebenen Abstand des Führungsschlitzes zur Grundfläche der entsprechenden Vertiefung und somit die mit der jeweiligen Halteeinrichtung erzielbare Dicke der herzustellenden Knorpelscheibe angeben.

Bei Weiterbildungen dieser Ausführungsformen können die Kennzeichnungen Zahlen umfassen, die den jeweiligen vorgegebenen Abstand des Führungsschlitzes zur Grundfläche der entsprechenden Vertiefung und somit die mit der jeweiligen Halteeinrichtung erzielbare Dicke der herzustellenden Knorpelscheibe im metrischen Maßsystem, insbesondere in Millimetern, oder im zöllischen Maßsystem, insbesondere in Inch, angeben.

Alternativ oder ergänzend können die Kennzeichnungen aber auch graphische Darstellungen, insbesondere Skalenstriche, Punkte und dergleichen umfassen, die den jeweiligen vorgegebenen Abstand des Führungsschlitzes zur Grundfläche der entsprechenden Vertiefung und somit die mit der jeweiligen Halteeinrichtung erzielbare Dicke der herzustellenden Knorpelscheibe angeben.

In der Handhabung besonders günstig sind Varianten der oben beschriebenen Ausführungsformen, bei denen die Kennzeichnungen auf der die Vertiefungen tragenden Oberseite des Vorrichtungskörpers und/oder auf der der (oder den) Druckplatte(n) gegenüberliegenden Unterseite des Deckels angebracht sind.

Bei einer weiteren ergonomisch günstigen Ausführungsform der erfindungsgemäßen Schneidevorrichtung sind auf der den Vertiefungen gegenüberliegenden Unterseite des Vorrichtungskörpers und/oder auf der den Druckplatten gegenüberliegenden Unterseite des Deckels konvexe und/oder konkave Griffhilfen angebracht, die eine Orientierungshilfe zur Ausübung eines Druckes auf das jeweilige Zentrum der Vorsprünge bieten.

Auf der den Druckplatten gegenüberliegenden Unterseite des Deckels kann bei Ausführungsformen der Erfindung eine randseitig umlaufende Umrandung vorgesehen sein.

Ebenso kann bei weiteren Ausführungsformen auf der Unterseite des Vorrichtungskörpers eine umlaufende Wandung vorgesehen sein, die einen oder mehrere Arbeitsräume umschließt, welche beispielsweise zur Vorbearbeitung eines Knorpelstücks vor dem Abschneiden der gewünschten Scheibe oder zur weiteren Bearbeitung der abgeschnittenen Knorpelscheibe dienen können.

Der Bearbeitung von abgeschnittenen Knorpelscheiben können auch runde und/oder ovale Templates unterschiedlichen Durchmessers dienen, welche bei Ausführungsformen der erfindungsgemäßen Schneidevorrichtung in einer Fläche der Schneidevorrichtung, insbesondere in einem der Arbeitsräume auf der den Vertiefungen gegenüber liegenden Unterseite des Vorrichtungskörpers und/oder auf der den Druckplatten gegenüber liegenden Unterseite des Deckels eingearbeitet sind.

Ganz besonders vorteilhaft sind Ausführungsformen der erfindungsgemäßen Schneidevorrichtung, bei denen in einer Fläche der Schneidevorrichtung, insbesondere auf der den Vertiefungen gegenüber liegenden Unterseite des Vorrichtungskörpers, vorzugsweise in einem Arbeitsraum, und/oder auf der den Druckplatten gegenüber liegenden Unterseite des Deckels eine Mess-Skala eingearbeitet ist, mit deren Hilfe die zu verarbeitenden Knorpelstücke und/oder die abgeschnittenen Knorpelscheiben problemlos vermessen werden können. Auch können hier runde oder ovale Templates unterschiedlichen Durchmessers zur Feinbearbeitung der Knorpelstücke eingearbeitet sein.

Ähnlich wie bei dem in US 2010/0286693 A1 beschriebenen Knorpelschneider zeichnet sich eine Klasse von vorteilhaften Ausführungsformen der erfindungsgemäßen Schneidevorrichtung dadurch aus, dass mindestens eine zweite Halteeinrichtung vorgesehen ist, die einen zweiten Arbeitsabschnitt mit einer auf der Oberseite des Vorrichtungskörpers angeordneten zweiten Vertiefung aufweist, welche von einem zweiten Begrenzungssteg ganz oder teilweise umrandet ist, dass der zweite Arbeitsabschnitt am Verbindungsabschnitt unmittelbar starr anhängt, dass am Gegenstück zum Verbindungsabschnitt ein zweiter Druckabschnitt unmittelbar starr anhängt, dass der zweite Druckabschnitt eine federnd im zweiten Druckabschnitt gehaltene zentrale zweite Druckplatte aufweist, welche im Betriebszustand der vom zweiten Begrenzungssteg ganz oder teilweise umrandeten zweiten Vertiefung gegenüberliegt, und dass die Abschnitte geometrisch derart gestaltet sind, dass im Betriebszustand der zweite Druckabschnitt der zweiten Vertiefung des zweiten Arbeitsabschnitts mit einer durch die geometrische Formgebung des Verbindungsabschnitts sowie dessen Gegenstücks definierten, im Wesentlichen gleichmäßigen zweiten Distanz d_{b} gegenüberliegt, so dass ein zwischen dem zweiten Arbeitsabschnitt und dem zweiten Druckabschnitt verlaufender zweiter Führungsschlitz um Einschieben einer Schneidklinge frei bleibt.

Besonders einfache und kompakte Weiterbildungen dieser Klasse von Ausführungsformen der erfindungsgemäßen Schneidevorrichtung besitzen eine dritte Halteeinrichtung, die einen dritten Arbeitsabschnitt mit einer auf der Oberseite des Vorrichtungskörpers angeordneten dritten Vertiefung aufweist, welche von einem dritten Begrenzungssteg ganz oder teilweise umrandet ist, wobei der dritte Arbeitsabschnitt am Verbindungsabschnitt unmittelbar starr anhängt, wobei am Gegenstück zum Verbindungsabschnitt ein dritter Druckabschnitt unmittelbar starr anhängt, wobei der dritte Druckabschnitt eine federnd im dritten Druckabschnitt gehaltene zentrale dritte Druckplatte aufweist, welche im Betriebszustand der vom dritten Begrenzungssteg ganz oder teilweise umrandeten dritten Vertiefung gegenüberliegt, und wobei die Abschnitte geometrisch derart gestaltet sind, dass im Betriebszustand der dritte Druckabschnitt der dritten Vertiefung des dritten Arbeitsabschnitts mit einer durch die geometrische Formgebung des Verbindungsabschnitts sowie dessen Gegenstücks definierten, im Wesentlichen gleichmäßigen dritten Distanz d_{c} gegenüberliegt, so dass ein zwischen dem dritten Arbeitsabschnitt und dem dritten Druckabschnitt verlaufender zweiter Führungsschlitz zum Einschieben einer Schneidklinge frei bleibt, und wobei die drei Halteeinrichtungen relativ zueinander vorzugsweise in Kreuzform angeordnet sind.

In den meisten Fällen ist es nämlich erfahrungsgemäß ausreichend, wenn für eine Mittelohroperation drei verschiedene Dicken der herzustellenden Knorpelscheiben zur Auswahl stehen, um eine optimale Anpassung an die individuellen Gegebenheiten des Patienten durchführen zu können. Falls gleichwohl eine feinere Differenzierung erfolgen soll, können auch mehrere dieser Ausführungsformen nebeneinander verwendet werden, wobei dann jede einzelne Schneidevorrichtung einen anderen Dickenbereich abdecken sollte und eine Feinauswahl aufgrund der angebotenen drei verschiedenen Dicken im ausgewählten Dickenbereich erfolgen kann.

Eine Klasse von Ausführungsformen zeichnet sich dadurch aus, dass die seitlichen Begrenzungsstege jeweils einstückig umlaufend um die Vertiefungen gestaltet sind.

Bei einer dazu alternativen Klasse von Ausführungsformen sind die seitlichen Begrenzungsstege jeweils als mehrere Einzelstege gestaltet, wie an sich aus der EP 0 483 567 B1 bekannt ist.

In der Praxis bewähren sich Ausführungsformen der erfindungsgemäßen Schneidevorrichtung, bei denen die Schneidklinge eine Messerklinge aus Metall ist, insbesondere eine Rasiermesser-Klinge, und die Schneidklinge in einer Messerhalterung gehalten wird, die vorzugsweise aus Kunststoff hergestellt ist.

Bei einfachen Weiterbildungen dieser Ausführungsformen kann die Messerhalterung einteilig ausgeführt sein und einen Schlitz zum Einschieben der Schneidklinge aufweisen.

In alternativen Weiterbildungen ist die Messerhalterung zweiteilig ausgeführt und zum Halten der Schneidklinge klappbar gestaltet, was ein Austauschen der Schneidklinge erleichtert.

Andere Weiterbildungen der oben beschriebenen Ausführungsformen sind dadurch gekennzeichnet, dass die Messerhalterung mindestens eine Fläche aufweist, in der runde und/oder ovale Templates, vorzugsweise unterschiedlichen Durchmessers, eingearbeitet sind. Vorzugsweise ist eine randseitig um die Messerhalterung umlaufende Umrandung vorgesehen, welche das Flächeträgheitsmoment und damit die Stabilität der Halterung erhöht.

Eine andere Weiterbildung schließlich zeichnet sich dadurch aus, dass die Messerhalterung mindestens eine, vorzugsweise mehrere Durchgangsöffnungen aufweist.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen.

Die einzelnen Merkmale können je einzeln für sich oder zu mehreren in beliebigen Kombinationen bei Varianten der Erfindung verwirklicht sein. Die gezeigten und beschriebenen Ausführungsformen sind nicht als abschließende Aufzählung zu verstehen, sondern haben vielmehr beispielhaften Charakter für die Schilderung der Erfindung.

### Detaillierte Beschreibung der Erfindung und Zeichnung

In der schematischen Zeichnung sind Ausführungsbeispiele der Erfindung dargestellt, welche in der nachfolgenden Beschreibung näher erläutert werden.

Im Einzelnen zeigen:
- Fig. 1: eine schematische räumliche Darstellung von der Oberseite einer Ausführungsform der erfindungsgemäßen Schneidevorrichtung mit drei kreuzförmig angeordneten Halteeinrichtungen;
- Fig. 2: eine Ansicht auf die Unterseite der Ausführungsform aus Fig. 1;
- Fig. 3: eine Ansicht auf die Ausführungsform von Fig. 1 mit auf den Vorrichtungskörper aufgesetztem Deckel und in den Führungsschlitz einer der Halteeinrichtungen eingeschobener Schneidklinge mit Messerhalterung;
- Fig. 4a: eine einfache Ausführungsform der erfindungsgemäßen Schneidevorrichtung mit einer einzigen Halteeinrichtung kurz vor dem Klappen des Deckels auf den Vorrichtungskörper; und
- Fig. 4b: eine Ansicht auf die Oberseite des Deckels der Ausführungsform aus Fig. 4a.

Die in den Figuren der Zeichnung schematisch räumlich dargestellten Ausführungsformen der erfindungsgemäßen medizinischen **Schneidevorrichtung 10; 20** dienen zur Herstellung dünner Knorpelscheiben aus einem größeren Knorpelstück und sind aus einem sterilisierbaren Material, vorzugsweise aus sterilisierbarem Kunststoff und insbesondere in einem Spritzgussverfahren hergestellt. Sie umfassen einen **Vorrichtungskörper 11; 21** und einen **Deckel 12**; **22**, wobei eine erste Halteeinrichtung vorgesehen ist, die einen **ersten Arbeitsabschnitt 11a'; 21'** mit einer auf der Oberseite des Vorrichtungskörpers 11; 21 angeordneten **ersten Vertiefung 13a** aufweist, welche von einem **ersten Begrenzungssteg 14a** ganz - sowie bei in der Zeichnung nicht dargestellten Ausführungsformen wahlweise auch jeweils durch mehrere Einzeistege nur teilweise - umrandet ist.

Erfindungsgemäß sind die Schneidevorrichtungen 10; 20 dadurch gekennzeichnet, dass der Vorrichtungskörper 11; 21 einen **Verbindungsabschnitt 19'; 29'** aufweist, an welchem der erste Arbeitsabschnitt 11a'; 21' unmittelbar starr anhängt, dass der Deckel 12; 22 ein **Gegenstück 19"; 29"** zum Verbindungsabschnitt 19'; 29' aufweist, an welchem ein erster **Druckabschnitt 11a"; 21"** unmittelbar starr anhängt, dass der erste Druckabschnitt 11a"; 21" eine federnd im ersten Druckabschnitt 11a"; 21" gehaltene zentrale erste **Druckplatte 12a"; 22"** aufweist, welche in einem Betriebszustand zur Herstellung dünner Knorpelscheiben der vom ersten Begrenzungssteg 14a ganz oder teilweise umrandeten ersten Vertiefung 13a gegenüber liegt, und dass die Abschnitte 11a', 11a", 19', 19"; 21', 21", 29', 29" geometrisch derart gestaltet sind, dass im Betriebszustand das Gegenstück 19"; 29" zum Verbindungsabschnitt 19'; 29' arretierbar auf dem Verbindungsabschnitt 19'; 29' aufliegt und der erste Druckabschnitt 11a"; 21" der ersten Vertiefung 13a des ersten Arbeitsabschnitts 11a'; 21' mit einer durch die geometrische Formgebung des Verbindungsabschnitts 19'; 29' sowie dessen Gegenstücks 19"; 29" definierten, im Wesentlichen gleichmäßigen ersten Distanz dₐ gegenüberliegt, so dass ein zwischen dem ersten Arbeitsabschnitt 11a'; 21' und dem ersten Druckabschnitt 11a"; 21" verlaufender erster **Führungsschlitz 17a** zum Einschieben einer **Schneidklinge 38** frei bleibt.

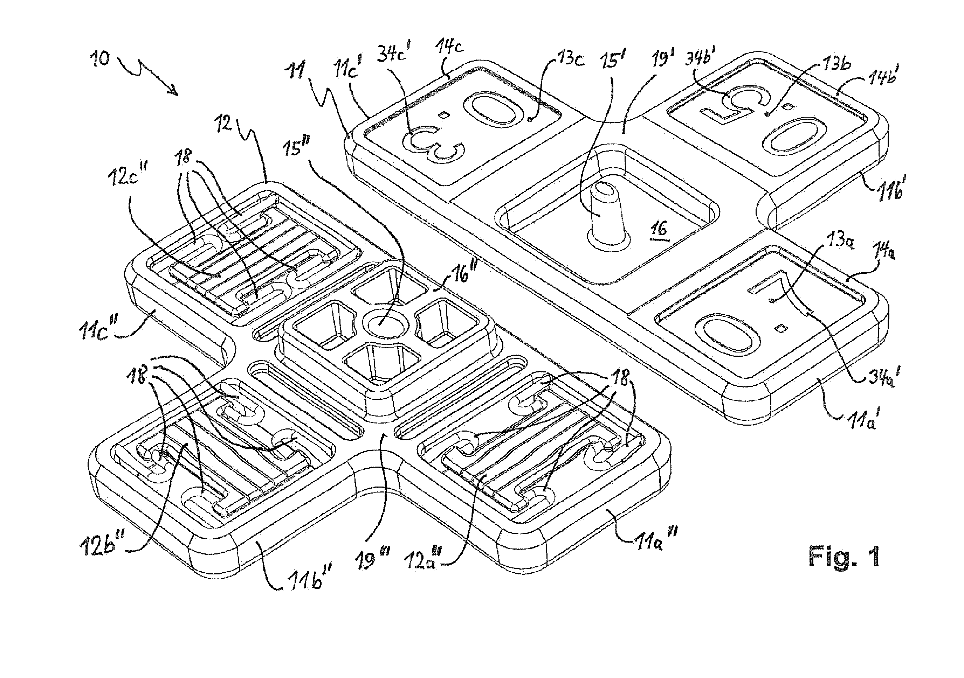

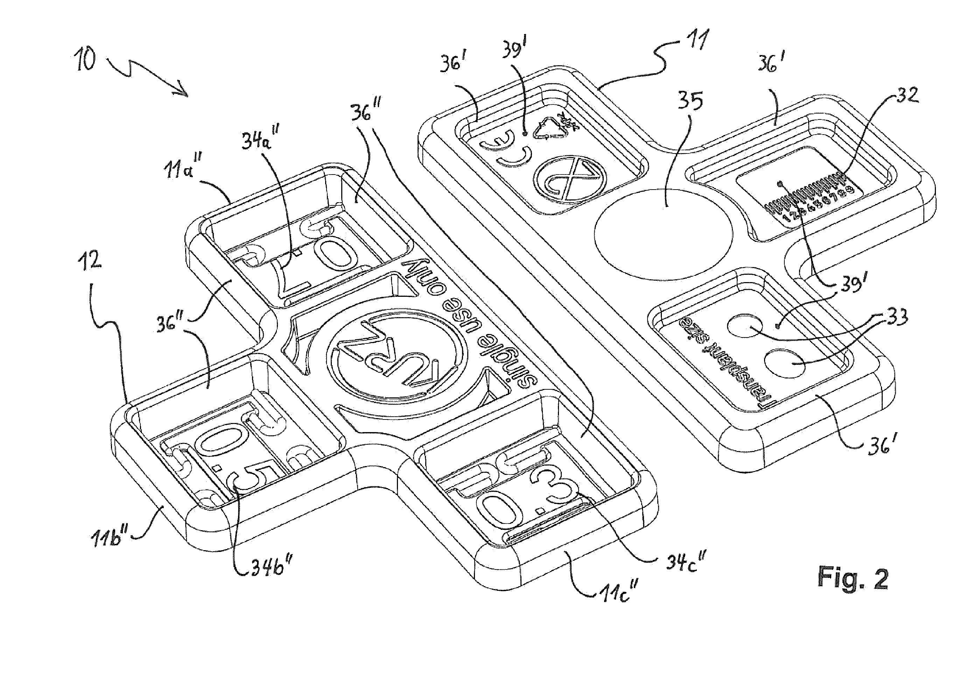

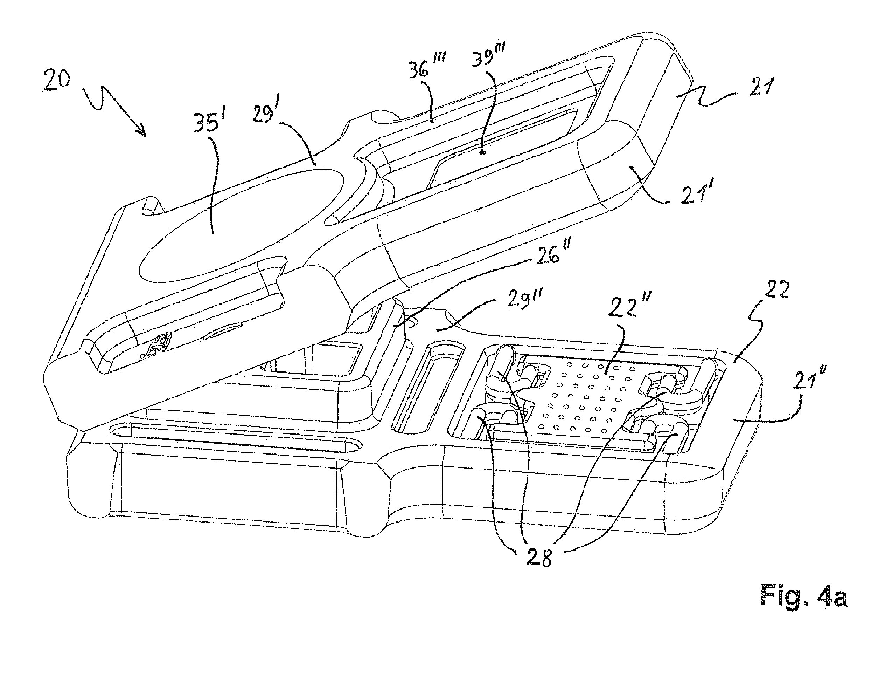

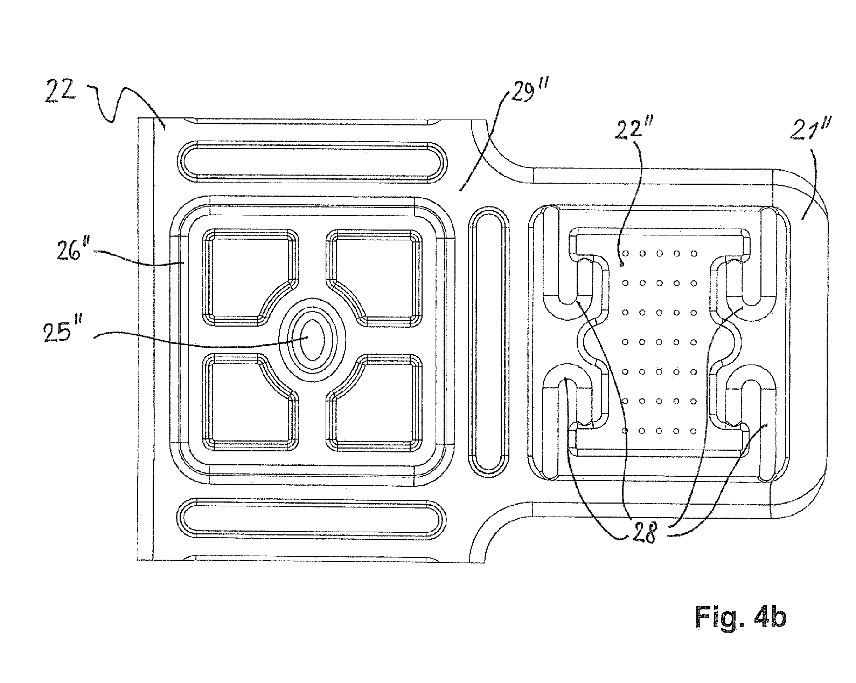

In den Figuren 4a und 4b ist eine Ausführungsform mit lediglich einer einzigen Halteeinrichtung dargestellt. Außer dieser ersten Halteeinrichtung zum Haltern des Knorpelstücks beim Zuschneiden einer Knorpelscheibe können auch eine zweite Halteeinrichtung und im Ausführungsbeispiel der Figuren 1 bis 3 auch noch eine dritte sowie bei in der Zeichnung nicht dargestellten Ausführungsformen der Erfindung sogar noch weitere Halteeinrichtungen vorgesehen sein. Die drei Halteeinrichtungen bei der gezeigten Ausführungsform gemäß den Figuren 1 bis 3 sind aus ergonomischen Gründen relativ zueinander in Kreuzform angeordnet

Die weiteren Halteeinrichtungen der Ausführungsform nach den Figuren 1 bis 3 umfassen jeweils ebenfalls einen **Arbeitsabschnitt 11b', 11c'** mit einer auf der Oberseite des Vorrichtungskörpers 11 angeordneten **Vertiefung 13b, 13c,** welche von einem **Begrenzungssteg 14b, 14c** ganz oder teilweise umrandet ist. Die Arbeitsabschnitte 11b', 11c' hängen jeweils am Verbindungsabschnitt 19' unmittelbar starr an. Am Gegenstück 19" zum Verbindungsabschnitt 19' hängen jeweils die **Druckabschnitte 11b", 11c"** an. Sie weisen wiederum federnd im jeweiligen Druckabschnitt 11b", 11c" gehaltene **zentrale Druckplatten 12b", 12c"** auf. Die Abschnitte 11b', 11b", 11c', 11c" sowie 19' und 19" sind geometrisch derart gestaltet, dass im zusammengeklappten Betriebszustand die Druckabschnitte 11b", 11c" der Vertiefung 13b, 13c des jeweils entsprechenden Arbeitsabschnitts 11b', 11c' mit einer durch die geometrische Formgebung des Verbindungsabschnitts 19' sowie dessen Gegenstücks 19" definierten, im Wesentlichen gleichmäßigen Distanz d_{b} beziehungsweise d_{c} gegenüberliegt, so dass ein zwischen dem Arbeitsabschnitt 11b', 11c' und dem Druckabschnitt 11b", 11c" verlaufender **Führungsschlitz 17b, 17c** zum Einschieben der Schneidklinge 38 frei bleibt.

Vorzugsweise ist bei der erfindungsgemäßen Schneidevorrichtung 10; 20 eine Arretiervorrichtung vorgesehen, die den Vorrichtungskörper 11; 21 und den Deckel 12; 22 in einem zusammengeklappten Betriebszustand in einer fixen relativen Position zueinander hält. Diese umfasst bei den in der Zeichnung dargestellten Ausführungsformen mindestens einen **Zapfen 15'** und mindestens ein **Nutenloch 15"; 25",** in welchen der Zapfen 15' arretierend eingesteckt werden kann.

Der Verbindungsabschnitt 19'; 29' und sein Gegenstück 19"; 29" sind geometrisch derart geformt, dass der Vorrichtungskörper 11; 21 und der Deckel 12; 22 nur in einer bestimmten vorgegebenen Richtung zusammensetzbar sind. Bei den in der Zeichnung gezeigten Ausführungsbeispielen weist der Verbindungsabschnitt 19'; 29' des Vorrichtungskörpers 11; 21 auf seiner die Vertiefungen 13a, 13b, 13c tragenden Oberseite einen **Aufnahmeraum 16'** auf, in welchen eine **Erhöhung 16"; 26"** auf der die Druckplatten 12a", 12b", 12c"; 22" tragenden Oberseite des Deckels 12; 22 im Gegenstück 19"; 29" passgenau eingeführt werden kann. Die den Aufnahmeraum 16' umschließende Wandung sowie die die Erhöhung 16"; 26" umgebende Wandung bilden jeweils ein Polygon gleicher Eckenzahl und Geometrie, bei den in der Zeichnung gezeigten Ausführungsformen insbesondere ein Quadrat. Günstig für die gewünschte Festlegung einer bestimmten Einbaurichtung ist auch eine Dreiecksform.

Die Druckplatten 12a", 12b", 12c"; 22" bei den in der Zeichnung dargestellten Ausführungsformen der erfindungsgemäßen Schneidevorrichtung 10; 20 sind mittels gekrümmten **Stegelementen 18; 28** in Ausnehmungen der Druckabschnitte 11a", 11b", 11c"; 21" federnd eingehängt.

Die Grundflächen der Vertiefungen 13a, 13b, 13c und die den Vertiefungen 13a, 13b, 13c in einem zusammengeklappten Betriebszustand von Vorrichtungskörper 11; 21 und Deckel 12; 22 gegenüber liegende Flächen der Druckplatten 12a", 12b", 12c"; 22" sind aufgeraut, geriffelt oder genoppt.

Weiter zeichnet sich die in den Figuren 1 bis 3 gezeigte Ausführungsform dadurch aus, dass an den Arbeitsabschnitten 11a', 11b', 11c' des Vorrichtungskörpers 11 und/oder an den entsprechenden Druckabschnitten 11a", 11b", 111c" **Kennzeichnungen 34a', 34b', 34c'** beziehungsweise **34a", 34b", 34c"** (hier in Form von Zahlenangaben) angebracht sind, die den durch die jeweilige Distanz dₐ, d_{b}, d_{c} vorgegebenen Abstand des Führungsschlitzes 17a, 17b, 17c zur Grundfläche der entsprechenden Vertiefung 13a, 13b, 13c und somit die mit der jeweiligen Halteeinrichtung erzielbare Dicke einer mittels der Schneidevorrichtung 10 herzustellenden Knorpelscheibe angeben - im vorliegenden Ausführungsbeispiel in Millimetern. Alternativ können die Kennzeichnungen die entsprechend Dickenmaßzahlen auch im zöllischen Maßsystem, insbesondere in Inch, angeben. Auch können die Kennzeichnungen alternativ oder ergänzend graphische Darstellungen, insbesondere Skalenstriche, Punkte und dergleichen umfassen.

In der Regel werden diese Kennzeichnungen 34a', 34b', 34c'; 34a", 34b", 34c" auf der die Vertiefungen 13a, 13b, 13c tragenden Oberseite des Vorrichtungskörpers 11 und/oder auf der den Druckplatten 12a", 12b", 12c" gegenüberliegenden Unterseite des Deckels 12 angebracht sein.

Außerdem sind auf der den Druckplatten 12a", 12b", 12c"; 22" gegenüberliegenden Unterseite des Deckels 12; 22 und/oder auf der den Vertiefungen 13a, 13b, 13c gegenüber liegenden Unterseite des Vorrichtungskörpers 11 konvexe und/oder konkave **Griffhilfen 35; 35'** sowie vorzugsweise auch eine randseitig umlaufende **Umrandung 36"; 36"; 36"'** angebracht. Die Wandungen 36'; 36"' des Vorrichtungskörpers 11; 21 umschließen **Arbeitsräume 39"; 39"'.**

In einer Fläche der Schneidevorrichtung 10 - im gezeigten Ausführungsbeispiel in einem Arbeitsraum 39' - auf der den Vertiefungen 13a, 13b, 13c gegenüber liegenden Unterseite des Vorrichtungskörpers 11 - sind runde sowie ovale **Templates 33** unterschiedlichen Durchmessers zur weiteren Bearbeitung der hergestellten Knorpelscheiben sowie eine **Mess-Skala 32** zur Vermessung der bearbeiteten Knorpelscheiben eingearbeitet.

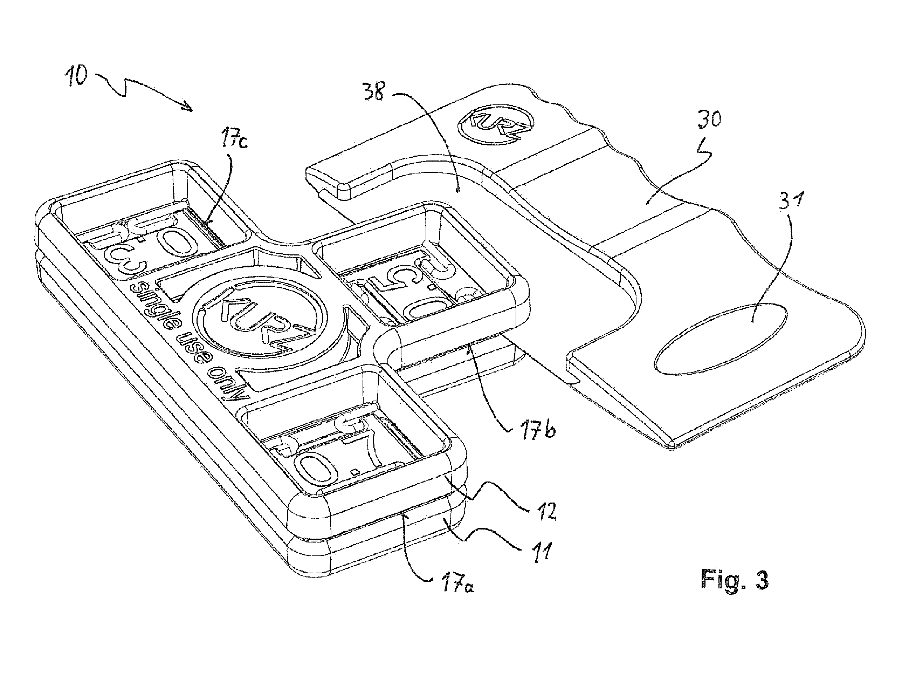

Die Schneidklinge 38 wird in der Regel eine Messerklinge aus Metall sein, insbesondere eine Rasiermesser-Klinge. Bei der in Fig. 3 beispielhaft dargestellten Ausführungsform ist die Schneidklinge 38 in einer einteiligen, vorzugsweise aus Kunststoff hergestellten **Messerhalterung 30** gehalten, die einen Schlitz zum Einschieben der Schneidklinge 38 aufweist. Bei in der Zeichnung nicht dargestellten Ausführungsformen der erfindungsgemäßen Schneidevorrichtung kann die Messerhalterung 30 auch anders, beispielsweise zweiteilig ausgeführt und zum Halten der Schneidklinge 38 klappbar gestaltet sein. Die Messerhalterung 30 kann **Durchgangsöffnungen 31** aufweisen, die beispielsweise rund und/oder oval gestaltet sein können.

## Patentansprüche

1. Medizinische Schneidevorrichtung (10; 20) zur Herstellung dünner Knorpelscheiben mit einem Vorrichtungskörper (11; 21) und einem Deckel (12; 22), wobei eine erste Halteeinrichtung vorgesehen ist, die einen ersten Arbeitsabschnitt (11a'; 21') mit einer auf der Oberseite des Vorrichtungskörpers (11; 21) angeordneten ersten Vertiefung (13a) aufweist, welche von einem ersten Begrenzungssteg (14a) ganz oder teilweise umrandet ist, und wobei die Schneidevorrichtung (10; 20) aus einem sterilisierbaren Material hergestellt ist,
wobei
der Vorrichtungskörper (11; 21) einen Verbindungsabschnitt (19'; 29') aufweist, an welchem der erste Arbeitsabschnitt (11a'; 21') unmittelbar starr anhängt,
wobei
der Deckel (12; 22) ein Gegenstück (19"; 29") zum Verbindungsabschnitt (19'; 29') aufweist, an welchem ein erster Druckabschnitt (11a"; 21") unmittelbar starr anhängt,
und wobei
die Abschnitte (11a", 11a", 19', 19"; 21', 21", 29', 29") geometrisch derart gestaltet sind, dass im Betriebszustand das Gegenstück (19"; 29") zum Verbindungsabschnitt (19'; 29') arretierbar auf dem Verbindungsabschnitt (19'; 29') aufliegt und der erste Druckabschnitt (11a"; 21") der ersten Vertiefung (13a) des ersten Arbeitsabschnitts (11a'; 21') mit einer durch die geometrische Formgebung des Verbindungsabschnitts (19'; 29') sowie dessen Gegenstücks (19"; 29") definierten, im Wesentlichen gleichmäßigen ersten Distanz dₐ gegenüberliegt, so dass ein zwischen dem ersten Arbeitsabschnitt (11a'; 21') und dem ersten Druckabschnitt (11a"; 21") verlaufender erster Führungsschlitz (17a) zum Einschieben einer Schneidklinge (38) frei bleibt,
**dadurch gekennzeichnet,**
**dass** der erste Druckabschnitt (11a"; 21 ") eine federnd im ersten Druckabschnitt (11a";21 ") gehaltene zentrale erste Druckplatte (12a"; 22") aufweist, welche in einem Betriebszustand zur Herstellung dünner Knorpelscheiben der vom ersten Begrenzungssteg (14a) ganz oder teilweise umrandeten ersten Vertiefung (13a) gegenüberliegt.

2. Schneidevorrichtung nach Anspruch 1, **dadurch gekennzeichnet, dass** eine Arretiervorrichtung vorgesehen ist, die den Vorrichtungskörper (11; 21) und den Deckel (12; 22) in einem zusammengeklappten Betriebszustand in einer fixen relativen Position zueinander hält.

3. Schneidevorrichtung nach Anspruch 2, **dadurch gekennzeichnet, dass** die Arretiervorrichtung mindestens einen Zapfen (15') und mindestens ein Nutenloch (15"; 25") umfasst, in welchen der Zapfen (15') arretierend eingesteckt werden kann.

4. Schneidevorrichtung nach einem der vorhergehenden Ansprüche, **dadurch gekennzeichnet, dass** der Verbindungsabschnitt (19'; 29') und sein Gegenstück (19"; 29") geometrisch derart geformt sind, dass der Vorrichtungskörper (11; 21) und der Deckel (12; 22) nur in einer bestimmten vorgegebenen Richtung zusammensetzbar sind.

5. Schneidevorrichtung nach Anspruch 4, **dadurch gekennzeichnet, dass** der Verbindungsabschnitt (19'; 29') des Vorrichtungskörpers (11; 21) auf seiner die Vertiefungen (13a, 13b, 13c) tragenden Oberseite einen Aufnahmeraum (16') aufweist, in welchen eine Erhöhung (16"; 26") auf der die Druckplatten (12a", 12b", 12c"; 22") tragenden Oberseite des Deckels (12; 22) im Gegenstück (19"; 29") passgenau eingeführt werden kann.

6. Schneidevorrichtung nach Anspruch 5, **dadurch gekennzeichnet, dass** die den Aufnahmeraum (16') umschließende Wandung sowie die die Erhöhung (16"; 26") umgebende Wandung jeweils ein Polygon gleicher Eckenzahl und Geometrie, vorzugsweise ein Dreieck oder ein Viereck, insbesondere ein Quadrat bilden.

7. Schneidevorrichtung nach einem der vorhergehenden Ansprüche, **dadurch gekennzeichnet, dass** die Druckplatten (12a", 12b", 12c"; 22") federnd, insbesondere mittels vorzugsweise gekrümmten Stegelementen (18; 28), in Ausnehmungen der Druckabschnitte (11a", 11b", 11c"; 21") eingehängt sind.

8. Schneidevorrichtung nach einem der vorhergehenden Ansprüche, **dadurch gekennzeichnet, dass** die Grundflächen der Vertiefungen (13a, 13b, 13c) und/oder die den Vertiefungen (13a, 13b, 13c) in einem zusammengeklappten Betriebszustand von Vorrichtungskörper (11; 21) und Deckel (12; 22) gegenüber liegende Flächen der Druckplatten (12a", 12b", 12c"; 22") aufgeraut, geriffelt oder genoppt sind.

9. Schneidevorrichtung nach einem der vorhergehenden Ansprüche, **dadurch gekennzeichnet, dass** an den Arbeitsabschnitten (11a', 11b', 11c'; 21') des Vorrichtungskörpers (11; 21) und/oder an den entsprechenden Druckabschnitten (11a", 11b", 11c"; 21") des Deckels (12; 22), Kennzeichnungen (34a', 34b', 34c'; 34a", 34b", 34c") angebracht sind, die den durch die jeweilige Distanz dₐ, d_{b}, d_{c} vorgegebenen Abstand des Führungsschlitzes (17a, 17b, 17c) zur Grundfläche der entsprechenden Vertiefung (13a, 13b, 13c) und somit die mit der jeweiligen Halteeinrichtung erzielbare Dicke einer mittels der Schneidevorrichtung (10; 20) herzustellenden Knorpelscheibe angeben.

10. Schneidevorrichtung nach einem der vorhergehenden Ansprüche, **dadurch gekennzeichnet, dass** auf der den Vertiefungen (13a, 13b, 13c) gegenüberliegenden Unterseite des Vorrichtungskörpers (11; 21) und/oder auf der den Druckplatten (12a", 12b", 12c"; 22") gegenüberliegenden Unterseite des Deckels (12; 22) konvexe und/oder konkave Griffhilfen (35; 35') angebracht sind.

11. Schneidevorrichtung nach einem der vorhergehenden Ansprüche, **dadurch gekennzeichnet, dass** auf der den Vertiefungen (13a, 13b, 13c) gegenüberliegenden Unterseite des Vorrichtungskörpers (11; 21) eine randseitig umlaufende Umrandung (36'; 36") vorgesehen ist, welche Arbeitsräume (39'; 39"') umschließt.

12. Schneidevorrichtung nach Anspruch 11, **dadurch gekennzeichnet, dass** in einer Fläche der Schneidevorrichtung (10; 20), insbesondere in einem der Arbeitsräume (39'; 39"'), eine Mess-Skala (32) und/oder runde oder ovale Templates (33) unterschiedlichen Durchmessers eingearbeitet sind.

13. Schneidevorrichtung nach einem der vorhergehenden Ansprüche, **dadurch gekennzeichnet, dass** mindestens eine zweite Halteeinrichtung vorgesehen ist, die einen zweiten Arbeitsabschnitt (11b') mit einer auf der Oberseite des Vorrichtungskörpers (11) angeordneten zweiten Vertiefung (13b) aufweist, welche von einem zweiten Begrenzungssteg (14b) ganz oder teilweise umrandet ist, dass der zweite Arbeitsabschnitt (11b') am Verbindungsabschnitt (19') unmittelbar starr anhängt, dass am Gegenstück (19") zum Verbindungsabschnitt (19') ein zweiter Druckabschnitt (11b") unmittelbar starr anhängt, dass der zweite Druckabschnitt (11b") eine federnd im zweiten Druckabschnitt (11b") gehaltene zentrale zweite Druckplatte (12b") aufweist, welche im Betriebszustand der vom zweiten Begrenzungssteg (14b) ganz oder teilweise umrandeten zweiten Vertiefung (13b) gegenüberliegt, und dass die Abschnitte (11b', 11b", 19', 19") geometrisch derart gestaltet sind, dass im Betriebszustand der zweite Druckabschnitt (11b") der zweiten Vertiefung (13b) des zweiten Arbeitsabschnitts (11b') mit einer durch die geometrische Formgebung des Verbindungsabschnitts (19') sowie dessen Gegenstücks (19") definierten, im Wesentlichen gleichmäßigen zweiten Distanz d_{b} gegenüberliegt, so dass ein zwischen dem zweiten Arbeitsabschnitt (11b') und dem zweiten Druckabschnitt (11b") verlaufender zweiter Führungsschlitz (17b) zum Einschieben einer Schneidklinge (38) frei bleibt.

14. Schneidevorrichtung nach Anspruch 13, **dadurch gekennzeichnet, dass** eine dritte Halteeinrichtung vorgesehen ist, die einen dritten Arbeitsabschnitt (11c') mit einer auf der Oberseite des Vorrichtungskörpers (11) angeordneten dritten Vertiefung (13c) aufweist, welche von einem dritten Begrenzungssteg (14c) ganz oder teilweise umrandet ist, dass der dritte Arbeitsabschnitt (11c') am Verbindungsabschnitt (19') unmittelbar starr anhängt, dass am Gegenstück (19") zum Verbindungsabschnitt (19') ein dritter Druckabschnitt (11c") unmittelbar starr anhängt, dass der dritte Druckabschnitt (11c") eine federnd im dritten Druckabschnitt (11c") gehaltene zentrale dritte Druckplatte (12c") aufweist, welche im Betriebszustand der vom dritten Begrenzungssteg (14c) ganz oder teilweise umrandeten dritten Vertiefung (13c) gegenüberliegt, und dass die Abschnitte (11c', 11c", 19', 19") geometrisch derart gestaltet sind, dass im Betriebszustand der dritte Druckabschnitt (11c") der dritten Vertiefung (13c) des dritten Arbeitsabschnitts (11c') mit einer durch die geometrische Formgebung des Verbindungsabschnitts (19') sowie dessen Gegenstücks (19") definierten, im Wesentlichen gleichmäßigen dritten Distanz d_{c} gegenüberliegt, so dass ein zwischen dem dritten Arbeitsabschnitt (11c') und dem dritten Druckabschnitt (11c") verlaufender dritter Führungsschlitz (17c) zum Einschieben einer Schneidklinge (38) frei bleibt, und dass die drei Halteeinrichtungen relativ zueinander vorzugsweise in Kreuzform angeordnet sind.

## Claims

1. A medical cutting tool (10; 20) for producing thin slices of cartilage, said tool comprising a tool body (11; 21) and a cover (12; 22), in which case a first holding device is provided, which comprises a first work section (11a'; 21') having a first recess (13a) which is located on the top side of the tool body (11; 21) and is entirely or partially bordered by a first delimiting rim (14a); the cutting tool (10; 20) is made of a sterilizable material; the tool body (11; 21) has a connecting portion (19'; 29'), to which the first work section (11a'; 21') is directly and rigidly attached; the cover (12; 22) has a counterpart (19"; 29") for the connecting portion (19'; 29'), to which counterpart (19"; 29") a first pressure section (11a"; 21") is directly and rigidly attached; the parts (11a', 11a", 19', 19", 21', 21 ", 29', 29") are geometrically configured in such a way that, in the operating position, the counterpart (19"; 29") for the connecting portion (19'; 29') can be secured so as to rest on said connecting portion (19'; 29') and the first pressure section (11a"; 21") is located opposite the first recess (13a) of the first work section (11a'; 21') at a substantially constant first distance dₐ, as defined by the geometric shaping of the connecting portion (19'; 29') and of its counterpart (19"; 29"), so that a first guide slot (17a), extending between the first work section (11a'; 21') and the first pressure section (11a"; 21 "), remains clear for the insertion of a cutting blade (38),
**characterized in that**
the first pressure section (11a"; 21 ") includes a central first pressure plate (12a"; 22") which is resiliently secured in said first pressure section (11a"; 21 ") and which, in an operating position for producing thin slices of cartilage, is located opposite the first recess (13a) which is entirely or partially bordered by the first delimiting rim (14a).

2. A cutting tool according to Claim 1, **characterized in that** a locking device is provided which, in the closed operating position, holds the tool body (11; 21) and cover (12; 22) in a fixed position relative to one another.

3. A cutting tool according to Claim 2, **characterized in that** the locking device comprises at least one peg (15') and at least one grooved hole (15"; 25"), into which the peg (15') can be inserted in a locking capacity.

4. A cutting tool according to one of the preceding claims, **characterized in that** the connecting portion (19'; 29') and its counterpart (19"; 29") are geometrically formed in such a way that the tool body (11; 21) and cover (12; 22) can only be assembled in a certain predetermined direction.

5. A cutting tool according to Claim 4, **characterized in that** the connecting portion (19'; 29') of the tool body (11; 21) has, on its top side bearing the recesses (13a, 13b, 13c), a receiving space (16'), into which a raised portion (16"; 26"), located in the counterpart (19"; 29") on the top side of the cover (12; 22) bearing the pressure plates (12a", 12b", 12c"; 22"), can be inserted with precise fit.

6. A cutting tool according to Claim 5, **characterized in that** the wall surrounding the receiving space (16') and the wall surrounding the raised portion (16"; 26") each form a polygon having the same number of angles and the same geometry, preferably a triangle or a rectangle, and in particular a square.

7. A cutting tool according to one of the preceding claims, **characterized in that** the pressure plates (12a", 12b", 12c"; 22") are resiliently suspended, preferably in particular by means of curved bar elements (18; 28), in recesses in the pressure sections (1a", 11b", 11c"; 21 ").

8. A cutting tool according to one of the preceding claims, **characterized in that** the bottom surfaces of the recesses (13a, 13b, 13c) and/or the surfaces of the pressure plates (12a", 12b", 12c"; 22") facing the recesses (13a, 13b, 13c) in the closed operating position of tool body (11; 21) and cover (12; 22) are roughened, grooved or knubbed.

9. A cutting tool according to one of the preceding claims, **characterized in that** there are markings (34a', 34b', 34c'; 34a", 34b", 34c") on the work sections (11a', 11b', 11c'; 21') of the tool body (11; 21) and/or on the corresponding pressure sections (11a", 11b", 11c"; 21") of the cover (12; 22), and said markings (34a', 34b', 34c'; 34a", 34b", 34c") indicate the distance, predetermined by the respective distance dₐ, d_{b}, d_{c}, of the guide slot (17a, 17b, 17c) from the bottom surface of the corresponding recess (13a, 13b, 13c) and thus the thickness of a slice of cartilage achievable by means of the cutting tool (10; 20) with the respective holding device.

10. A cutting tool according to one of the preceding claims, **characterized in that** convex and/or concave gripping aids (35; 35') are provided on the underside of the tool body (11; 21), i.e. on the opposite side to the recesses (13a, 13b, 13c), and/or on the underside of the cover (12; 22), i.e. on the opposite side to the pressure plates (12a", 12b", 12c"; 22").

11. A cutting tool according to one of the preceding claims, **characterized in that**, provided on the underside of the tool body (11; 21), i.e. on the opposite side to the recesses (13a, 13b, 13c), there is a peripheral rim (36'; 36") surrounding work spaces (39'; 39"').

12. A cutting tool according to Claim 11, **characterized in that** a measurement scale (32) and/or round or oval templates (33) of different diameters are incorporated in one area of the cutting tool (10; 20), and in particular in one of the work spaces (39'; 39"').

13. A cutting tool according to one of the preceding claims, **characterized in that** at least a second holding device is provided, which comprises a second work section (11b') having a second recess (13b) which is located on the top side of the tool body (11) and is entirely or partially bordered by a second delimiting rim (14b), **in that** the second work section (11b') is directly and rigidly attached to the connecting portion (19'), **in that** a second pressure section (11b") is directly and rigidly attached to the counterpart (19") for the connecting portion (19'), **in that** the second pressure section (11b") has a second central pressure plate (12b"), which is resiliently secured in said second pressure section 116") and which, in the operating position, is located opposite the second recess (13b) which is entirely or partially bordered by the second delimiting rim (14b), and **in that** the parts (11b', 11b", 19', 19") are geometrically configured in such a way that, in the operating position, the second pressure section (11b") is located opposite the second recess (13b) of the second work section (11b') at a substantially constant second distance d_{b}, as defined by the geometric shaping of the connecting portion (19') and of its counterpart (19"), so that a second guide slot (17b), extending between the second work section (11b') and the second pressure section (11b"), remains clear for the insertion of a cutting blade (38).

14. A cutting tool according to Claim 13, **characterized in that** a third holding device is provided, which comprises a third work section (11c') having a third recess (13c) which is located on the top side of the tool body (11) and is entirely or partially bordered by a third delimiting rim (14c), **in that** the third work section (11c') is directly and rigidly attached to the connecting portion (19'), **in that** a third pressure section (11c") is directly and rigidly attached to the counterpart (19") for the connecting portion (19'), **in that** the third pressure section (11c") has a central third pressure plate (12c") which is resiliently secured in said third pressure section (11c") and which, in the operating position, is located opposite the third recess (13c) which is entirely or partially bordered by the third delimiting rim (14c), and **in that** the parts (11c', 11c", 19', 19") are geometrically configured in such a way that, in the operating position, the third pressure section (11c") is located opposite the third recess (13c) of the third work section (11c') at a substantially constant third distance d_{c}, as defined by the geometric shaping of the connecting portion (19') and of its counterpart (19"), so that a third guide slot (17c), extending between the third work section (11c') and the third pressure section (11c"), remains clear for the insertion of a cutting blade (38), and **in that** the three holding devices are preferably disposed relative to one another in the form of a cross.

## Revendications

1. Dispositif de coupe (10 ; 20) médical destiné à la fabrication de minces disques de cartilage avec un corps de dispositif (11 ; 21) et un couvercle (12 ; 22), dans lequel il est prévu un premier dispositif de retenue qui présente un premier tronçon de travail (11a' ; 21') avec une première dépression (13a), disposée sur le côté supérieur du corps de dispositif (11 ; 21), qui est bordée entièrement ou partiellement par une première nervure de délimitation (14a), et dans lequel le dispositif de coupe (10 ; 20) est réalisé dans un matériau stérilisable,
dans lequel
le corps de dispositif (11 ; 21) présente un tronçon de raccordement (19' ; 29') auquel est suspendu le premier tronçon de travail (11a' ; 21') directement de façon rigide,
dans lequel le couvercle (12 ; 22) présente une pièce antagoniste (19" ; 29") au tronçon de raccordement (19' ; 29') à laquelle est suspendu un premier tronçon de pression (11a" ; 21") directement de façon rigide,
et dans lequel les tronçons (11a', 11a", 19', 19" ; 21', 21", 29', 29") sont géométriquement constitués de telle sorte que, dans l'état de fonctionnement, la pièce antagoniste (19" ; 29") au tronçon de raccordement (19' ; 29') repose de façon blocable sur le tronçon de raccordement (19' ; 29'), et le premier tronçon de pression (11a" ; 21") est opposé à la première dépression (13a) du premier tronçon de travail (11a' ; 21'), avec une première distance dₐ essentiellement homogène définie par la mise en forme géométrique du tronçon de raccordement (19' ; 29') ainsi que de sa pièce antagoniste (19" ; 29") de telle sorte qu'une première fente de guidage (17a) s'étendant entre le premier tronçon de travail (11a' ; 21') et le premier tronçon de pression (11a" ; 21") demeure libre pour l'insertion d'une lame de coupe (38),
**caractérisé en ce que** le premier tronçon de pression (11a" ; 21") présente une première plaque de pression (12a" ; 22") centrale, retenue de façon élastique dans le premier tronçon de pression (11a" ; 21 "), qui, dans un état de fonctionnement pour la fabrication de minces disques de cartilage, est opposée à la première dépression (13a) bordée entièrement ou partiellement par la première nervure de délimitation (14a).

2. Dispositif de coupe selon la revendication 1, **caractérisé en ce qu'**il est prévu un dispositif de blocage qui retient le corps de dispositif (11 ; 21) et le couvercle (12 ; 22) dans un état de fonctionnement replié, dans une position relative fixe l'un par rapport à l'autre.

3. Dispositif de coupe selon la revendication 2, **caractérisé en ce que** le dispositif de blocage présente au moins un tourillon (15') et au moins un trou de rainure (15" ; 25") dans lesquels le tourillon (15') peut être enfiché de façon bloquée.

4. Dispositif de coupe selon l'une des revendications précédentes, **caractérisé en ce que** le tronçon de raccordement (19' ; 29') et sa pièce antagoniste (19" ; 29") sont géométriquement formés de telle sorte que le corps de dispositif (11 ; 21) et le couvercle (12 ; 22) ne peuvent être assemblés que dans une certaine direction prédéfinie.

5. Dispositif de coupe selon la revendication 4, **caractérisé en ce que** le tronçon de raccordement (19' ; 29') du corps de dispositif (11 ; 21) présente, sur son côté supérieur portant les dépressions (13a, 13b, 13c), un espace de réception (16') dans lequel une surélévation (16" ; 26") peut être introduite avec précision sur le côté supérieur du couvercle (12 ; 22) portant les plaques de pression (12a", 12b", 12c" ; 22"), dans la pièce antagoniste (19" ; 29").

6. Dispositif de coupe selon la revendication 5, **caractérisé en ce que** la paroi renfermant l'espace de réception (16') ainsi que la paroi entourant la surélévation (16" ; 26") forment respectivement un polygone ayant un nombre d'angles et une géométrie identiques, de préférence un triangle ou un quadrilatère, en particulier un carré.

7. Dispositif de coupe selon l'une des revendications précédentes, **caractérisé en ce que** les plaques de pression (12a", 12b", 12c" ; 22") sont suspendues de façon élastique, en particulier au moyen d'éléments de nervure (18 ; 28) de préférence courbes, dans des creux des tronçons de pression (11a", 11b", 11c" ; 21").

8. Dispositif de coupe selon l'une des revendications précédentes, **caractérisé en ce que** les surfaces de base des dépressions (13a, 13b, 13c) et/ou les surfaces des plaques de pression (12a", 12b", 12c" ; 22") opposées aux dépressions (13a, 13b, 13c) dans un état de fonctionnement replié du corps de dispositif (11 ; 21) et du couvercle (12 ; 22) sont rendues rugueuses, cannelées ou pastillées.

9. Dispositif de coupe selon l'une des revendications précédentes, **caractérisé en ce que**, sur les tronçons de travail (11a', 11b', 11c' ; 21') du corps de dispositif (11 ; 21) et/ou sur les tronçons de pression (11a", 11b", 11c" ; 21") correspondants du couvercle (12 ; 22), il est apposé des signes distinctifs (34a', 34b', 34c' ; 34a", 34b", 34c") qui indiquent l'intervalle entre la fente de guidage (17a, 17b, 17c) et la surface de base de la dépression (13a, 13b, 13c) correspondante qui est prédéfini par la distance dₐ, d_{b}, d_{c} respective, et donc l'épaisseur, pouvant être obtenue avec le dispositif de retenue respectif, d'un disque de cartilage à fabriquer au moyen du dispositif de coupe (10 ; 20).

10. Dispositif de coupe selon l'une des revendications précédentes, **caractérisé en ce que**, sur le côté inférieur du corps de dispositif (11 ; 21) opposé aux dépressions (13a, 13b, 13c) et/ou sur le côté inférieur du couvercle (12 ; 22) opposé aux plaques de pression (12a", 12b", 12c" ; 22"), des aides à la préhension (35 ; 35') convexes et/ou concaves sont mises en place.

11. Dispositif de coupe selon l'une des revendications précédentes, **caractérisé en ce que**, sur le côté inférieur du corps de dispositif (11 ; 21) opposé aux dépressions (13a, 13b, 13c), il est prévu une bordure (36' ; 36") périphérique côté bord qui renferme des espaces de travail (39' ; 39"').

12. Dispositif de coupe selon la revendication 11, **caractérisé en ce que**, dans une surface du dispositif de coupe (10 ; 20), en particulier dans un des espaces de travail (39' ; 39"'), une échelle de mesure (32) et/ou des gabarits (33) ronds ou ovales de différents diamètres sont intégrés.

13. Dispositif de coupe selon l'une des revendications précédentes, **caractérisé en ce qu'**il est prévu au moins un deuxième dispositif de retenue qui présente un deuxième tronçon de travail (11 b') avec une deuxième dépression (13b), disposée sur le côté supérieur du cadre (11), qui est bordée entièrement ou partiellement par une deuxième nervure de délimitation (14b), **en ce que** le deuxième tronçon de travail (11b') est suspendu directement de façon rigide au tronçon de raccordement (19'), **en ce qu'**un deuxième tronçon de pression (11b") est suspendu directement de façon rigide à la pièce antagoniste (19') au tronçon de raccordement (19'), **en ce que** le deuxième tronçon de pression (11b") présente une deuxième plaque de pression (12b") centrale, retenue de façon élastique dans le deuxième tronçon de pression (11b"), qui, dans l'état de fonctionnement, est opposée à la deuxième dépression (13b) entièrement ou partiellement bordée par la deuxième nervure de délimitation (14b), et **en ce que** les tronçons (11b', 11b", 19', 19") sont géométriquement constitués de telle sorte que, dans l'état de fonctionnement, le deuxième tronçon de pression (11b") est opposé à la deuxième dépression (13b) du deuxième tronçon de travail (11b'), avec une deuxième distance d_{b} essentiellement homogène définie par la mise en forme géométrique du tronçon de raccordement (19') ainsi que de sa pièce antagoniste (19") de telle sorte qu'une deuxième fente de guidage (17b) s'étendant entre le deuxième tronçon de travail (11b') et le deuxième tronçon de pression (11b") demeure libre pour l'insertion d'une lame de coupe (38).

14. Dispositif de coupe selon la revendication 13, **caractérisé en ce qu'**il est prévu un troisième dispositif de retenue qui présente un troisième tronçon de travail (11c') avec une troisième dépression (13c), disposée sur le côté supérieur du corps de dispositif (11), qui est bordée entièrement ou partiellement par une troisième nervure de délimitation (14c), **en ce que** le troisième tronçon de travail (11c') est suspendu directement de façon rigide au tronçon de raccordement (19'), **en ce qu'**un troisième tronçon de pression (11c") est suspendu directement de façon rigide à la pièce antagoniste (19") au tronçon de raccordement (19'), **en ce que** le troisième tronçon de pression (11c") présente une troisième plaque de pression (12c") centrale, retenue de façon élastique dans le troisième tronçon de pression (11c"), qui, dans l'état de fonctionnement, est opposée à la troisième dépression (13c) bordée entièrement ou partiellement par la troisième nervure de délimitation (14c), et **en ce que** les tronçons (11c', 11c", 19', 19") sont géométriquement constitués de telle sorte que, dans l'état de fonctionnement, le troisième tronçon de pression (11c") est opposé à la troisième dépression (13c) du troisième tronçon de travail (11c'), avec une troisième distance d_{c} essentiellement homogène définie par la mise en forme géométrique du tronçon de raccordement (19') ainsi que de sa pièce antagoniste (19") de telle sorte qu'une troisième fente de guidage (17c) s'étendant entre le troisième tronçon de travail (1c') et le troisième tronçon de pression (11c") demeure libre pour l'insertion d'une lame de coupe (38), et **en ce que** les trois dispositifs de retenue sont disposés de préférence en forme de croix les uns par rapport aux autres.
